# EUROPEAN PATENT APPLICATION

(11) **EP 1 331 807 A1**
(43) Date of publication of application: **30.07.2003**
(21) Application number: 01976655.9
(22) Date of filing: 10.10.2001
(51) Int. Cl.: H04N 5/225

(54) **IRIS IMAGING DEVICE**

(30) Priority: 16.10.2000 JP 2000315438
(71) Applicant: MATSUSHITA ELECTRIC INDUSTRIAL CO., LTD., Kadoma-shi, Osaka 571-0050 (JP)
(72) Inventor: DOI, Makoto, Yokohama-shi, Kanagawa 226-0011 (JP); WADA, Jyoji, Yokohama-shi, Kanagawa 245-0003 (JP); IKOMA, Ken, Yokohama-shi, Kanagawa 225-0023 (JP); TAMURA, Kazushige, Yokohama-shi, Kanagawa 224-0006 (JP); NAKAIGAWA, Tomoyoshi, Yokohama-shi, Kanagawa 244-0802 (JP); OOI, Koji, Yokohama-shi, Kanagawa 226-0025 (JP); OOTSUNA, Yoshinori, Yokohama-shi, Kanagawa 230-0004 (JP); KOGANE, Haruo, Kawasaki-shi, Kanagawa 214-0038 (JP); KAWAZOE, Syuji, Yokohama-shi, Kanagawa 224-8539 (JP)
(74) Representative: Balsters, Robert
(86) International application number: JP0108890
(87) International publication number: WO02033963

(57) **Abstract**

The invention aims at providing low-cost iris image pickup apparatus for picking up iris images at a high accuracy.

In this invention, a telephotograph camera (22) and a range sensor (24) are mounted on a tilt table (20) that tilts upward/downward. A mirror for panning (23) and the range sensor (24) can be tilted rightward/leftward by a motor for panning (26) and a link mechanism (27). The image pickup direction of the telephotograph camera (22) is reflected by the mirror for panning (23) while the range sensor (24) is given a rotation angle double that of the mirror for panning (23) by the link mechanism (27). This matches the range direction and the image pickup direction thus allowing high-accuracy ranging. Accordingly only one wide angle camera (25) is required for picking up an iris image thus reducing the cost of the iris image pickup apparatus (10). The range sensor (24) is mounted on the tilt table (20) and makes ranging while tilted upward or downward thus expanding upward and downward the range of measuring the distance to the object.

## Description

### Technical Field

The present invention relates to an iris image pickup apparatus used in a security system, and in particular to an iris image pickup apparatus that tilt-controls a range sensor.

### Background Art

In a security system, a method is known to perform authentication by using the wave pattern of the iris of an individual. This method of using an iris, unlike a method using fingerprints, has an advantage of completing authentication by way of image pickup from a distance of an iris in a non-contact way. Thus the method is expected to be diffused in future. However, the image of an iris for use in authentication of an individual must be a clear image focused to improve the recognition rate and must be picked up while the person to be authenticated is at rest.

As conventional iris image pickup apparatus to satisfy the aforementioned restrictions, there is provided for example iris image pickup apparatus described in the Japanese Patent Publication No. 2000-23946.

This iris image pickup apparatus uses a pair of object person position recognition cameras to recognize the position of an object person and set the pickup position of the iris image pickup apparatus to the recognized object person position.

In the iris image pickup apparatus, a pair of object person position recognition cameras are provided and the object person position is recognized in accordance with the output of the object person position recognition cameras. Two cameras are required for such operation thus the cost of the iris image pickup apparatus is rather high.

Object person position recognition cameras to identify the object person position are fixed to the iris image pickup apparatus so that the range to recognize the object person position is limited within an object person position recognition camera.

The invention has been proposed in view of such a situation and aims at providing low-cost iris image pickup apparatus for picking up iris images at a high accuracy.

### Disclosure of the Invention

Iris image pickup apparatus to attain the aforementioned object is:
(1) An iris image pickup apparatus for picking up the iris of an object including
   a wide angle camera for picking up an image of an object,
   a range sensor for measuring the distance to the object,
   a telephotograph camera for picking up the iris of the object based on the image picked up by the wide angle camera and the range measured by the range sensor and
   a tilt table where the telephotograph camera and the range sensor are mounted with the image pickup direction of the telephotograph camera for the iris approximately matching the range direction of the range sensor for the object, the tilt table tilting upward/downward about the tilt rotation axis,
   wherein the range sensor measures the distance to the object with the tilting of the tilt table, and that
   wherein the telephotograph camera picks up the iris of the object based on the image picked up by the wide angle camera and the range measured by the range sensor.
   According to this iris image pickup apparatus, by providing a range sensor capable of detecting the distance to an object, it is possible to measure the distance to an object with a single wide angle camera (conventional object person position recognition camera) . This reduces the manufacturing cost of an iris image pickup apparatus.
   The range sensor is mounted on a tilt table and makes ranging while tilted upward or downward thus expanding upward and downward the range of measuring the distance to the object.
   Image pickup direction of the telephotograph camera coincides with the range direction of the range sensor so that it is possible to use the distance to the object detected by the range sensor for adjusting the focus of the telephotograph camera.
(2) The iris image pickup apparatus according to the invention is characterized in that the range sensor is positioned close to approximately beneath or above the telephotograph camera and that the range optical axis of the range sensor is parallel with the image pickup optical axis of the telephotograph camera.
   According to this iris image pickup apparatus, the range sensor is positioned close to approximately beneath or above the telephotograph camera and that the range optical axis of the range sensor is parallel with the image pickup optical axis of the telephotograph camera, so that it is possible to match the image pickup direction and the range direction.
   The telephotograph camera picks up the iris so that the ranging by a range sensor is made to approximately beneath the iris, that is, the cheek of an object or approximately above the iris, that is, the brow or forehead or an object. Both sections (cheek and brow (forehead)) is approximately flat and a range light irradiated by the range sensor is easily reflected toward the rage sensor direction, thus providing accurate ranging.
(3) The iris image pickup apparatus according to the invention is characterized in that the iris image pickup apparatus comprises swivel means for swiveling the image pickup direction by the telephotograph camera and the range direction by the range sensor to the direction approximately orthogonal to the operating direction of the tilt table.
   According to this iris image pickup apparatus, the range sensor may be panned as well as upward/downward by using the tilt table, as well as to a direction approximately orthogonal to the operating direction of the tilt table, that is clockwise and counterclockwise. This expands clockwise and counterclockwise the range of measuring the distance to the object
(4) The iris image pickup apparatus according to the invention is characterized in that the swivel means comprises a reflecting mirror for reflecting the image pickup direction by the telephotograph camera and the range direction by the range sensor and that the iris image pickup apparatus swivels the image pickup direction and the range direction to a direction approximately orthogonal to the operating direction of the tilt table.
   According to this iris image pickup apparatus, both the image pickup direction and the range direction are reflected by a reflecting mirror and swiveled by rotating the mirror. Thus it is possible to simultaneously rotate the image pickup direction and the range direction by rotating only the reflecting mirror, without rotating the telephotograph camera and the range sensor.
(5) The iris image pickup apparatus according to the invention is characterized in that the swivel means orients the range direction to an approximately center direction of the maximum swivel angle of the swivel means or to a predetermined direction while the range sensor is in standby state before detecting the object.
   According to this iris image pickup apparatus, the range direction is oriented to an approximately center direction of the maximum swivel angle of the swivel means or to a predetermined direction while the range sensor is in standby state. In case, for example, the object appears only in a certain direction, the swivel means does not swivel thus avoiding useless standby power consumption.
(6) The iris image pickup apparatus according to the invention is characterized in that the iris image pickup apparatus comprises a reflecting mirror for reflecting the image pickup direction by the telephotograph camera and
   link means for rotating the telephotograph camera to a direction approximately orthogonal to the operating direction of the tilt table at a rotation angle double the rotating angle of the reflecting mirror.
   According to this iris image pickup apparatus, it is possible to maintain both the image pickup direction and the range direction identical even when the directions are rotated by the link means.
(7) The iris image pickup apparatus according to the invention is characterized in that the link means orients the range direction to an approximately center direction of the maximum swivel angle of the swivel means or to a predetermined direction while the range sensor is in standby state before detecting the object.
   According to this iris image pickup apparatus, the range direction is oriented to an approximately center direction of the maximum swivel angle of the swivel means or to a predetermined direction while the range sensor is in standby state. In case, for example, the object appears only in a certain direction, the link means does not rotate thus avoiding useless standby power consumption.
(8) The iris image pickup apparatus according to the invention is characterized in that the ranging sensor comprises a stray light prevention hood on the sensor opening of the range sensor.

According to this iris image pickup apparatus, the stray light prevention hood reduces the stray light of lights being entered to the range sensor thus preventing ranging error by the range sensor.

### Brief Description of the Drawings

Fig. 1 is a general perspective view of iris image pickup apparatus of the embodiment.
Fig. 2 is a general front view of the iris image pickup apparatus.
Fig. 3 is an enlarged view of the periphery of a mirror for panning. Fig. 3 (a) is a top view of the periphery of the mirror for panning. Fig. 3(b) is a front view of the mirror for panning.
Fig. 4 shows a perspective view and a three-view drawing of a range sensor. Fig. 4(a) is a perspective view of the range sensor. Fig. 4(b) is a three-view drawing of the range sensor.
Fig. 5 shows the operation of a link mechanism. Fig. 5(a) shows the state the link mechanism has swiveled clockwise. Fig. 5(b) shows the state the link mechanism has swiveled counterclockwise.
Fig. 6 is an enlarged view of the periphery of a mirror for panning assumed when a range sensor is provided just beneath a telephotograph camera. Fig. 6(a) is a top view thereof. Fig. 6(b) is a front view thereof.
Fig. 7 is a circuit diagram of the electric system of the iris image pickup apparatus.
Fig. 8 is a flowchart showing the operating procedure for the iris image pickup apparatus.

In the figure, a numeral 10 represents iris image pickup apparatus, 11 a fixed table, 12, 13 iris lighting fixtures, 12a, 13a pan motors for lighting, 12b, 13b tilt motors for lighting, 14, 15 lighting fixtures for wide angle cameras, 16, 17 support plates, 20 a tilt table, 20a an axis, 20b an axis, 21 a motor for tilt, 22 a telephotograph camera, 23 a mirror for panning, 23a a mirror axis, 24 a range sensor, 24a a sensor axis, 24b sensor lenses, 24c a stray light prevention hood, 25 a wide angle camera, 26 a motor for panning, 26a a motor shaft, 27 a link mechanism, 271 a motor link member, 271a a first engaging section, 271b a second engaging section, 271c a link piece, 272 a stopper member, 273 a first mirror link member, 273a a first mirror pin, 273b a second mirror pin, 274 a second mirror link member, 275 a first sensor link member, 275a a first sensor pin, 275b a second sensor pin, 276 a second sensor link member, 30 a controller, and 31 a motor controller.

### Best Mode for Carrying Out the Invention

An iris image pickup apparatus according to the invention will be described referring to the drawings.

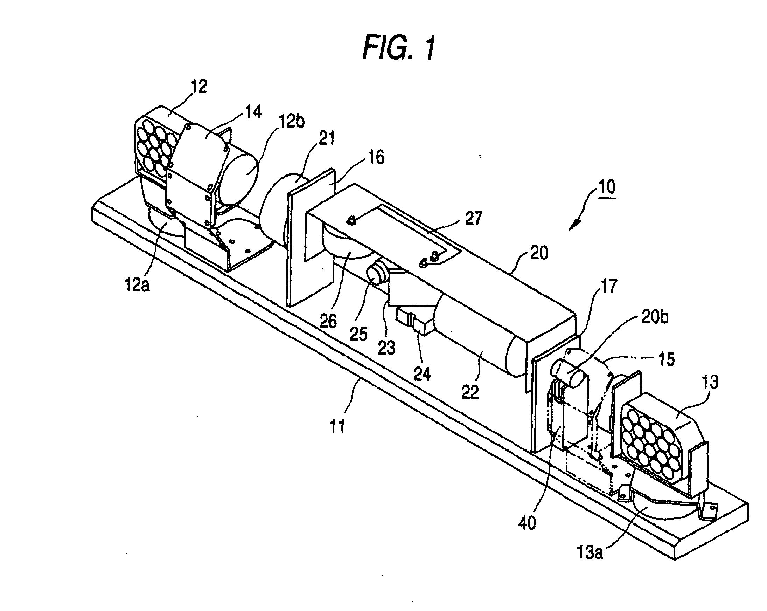

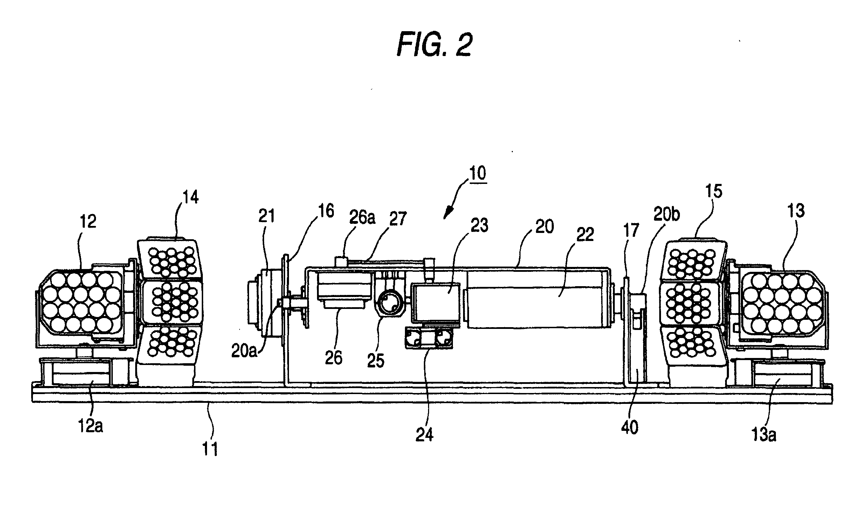

Fig. 1 is a general perspective view of an iris image pickup apparatus of the embodiment. Fig. 2 is a general front view of the iris image pickup apparatus.

Configuration of an iris image pickup apparatus 10 of this embodiment will be described first.

The iris image pickup apparatus 10 includes a longitudinal fixed table 11, each of the left and right ends of which are attached iris lighting fixtures 12, 13. Each iris lighting fixture 12, 13 includes a condensing lens for condensing and irradiating infrared rays on the iris and pan motors for lighting 12a, 13a and tilt motors 12b, 13b for lighting in order to orient the illuminating light in the direction of the iris.

Lighting fixtures for wide angle cameras 14, 15 are attached on the fixed table 11 in the inner direction seen from each iris lighting fixtures (in the center direction of the fixed table 11). The lighting fixtures for wide angle cameras 14, 15 has a great number of light emitting diodes attached on a mounting plate. Those light emitting diodes are not shown and only the mounting plate is shown in the perspective view of Fig. 2. The lighting fixtures for wide angle cameras 14, 15 have mounting plates oriented in approximately three directions, upward slanting direction, front direction and downward slanting direction in order to evenly illuminate a wide range with infrared rays.

A support plate 16 is erected on the fixed table 11 in the inner direction seen from the lighting fixture for wide angle cameras 14 (in the center direction of the fixed table 11). A support plate 17 is erected on the fixed table 11 in the inner direction seen from the lighting fixture for wide angle cameras 15 (in the center direction of the fixed table 11). A tilt table 20is attached between both support plates 16, 17.

The tilt table 20 includes axes 20a, 20b on the left and right respectively. Each axis 20a, 20b is respectively supported rotatably on the support plates 16, 17. One axis 20a is directly coupled to the rotation axis of the motor for tilt 21. On the other axis 20b is attached a damper 40 for controlling the rotation speed of the axis 20b.

The tilt table 20 mounts a telephotograph camera (narrow angle camera) 22, a mirror for panning 23 as a reflecting mirror, a range sensor 24, a wide angle camera 25, a motor for panning 26, and a link mechanism 27.

The motor for panning 26 and the link mechanism 27 as link means configure swivel means in this embodiment.

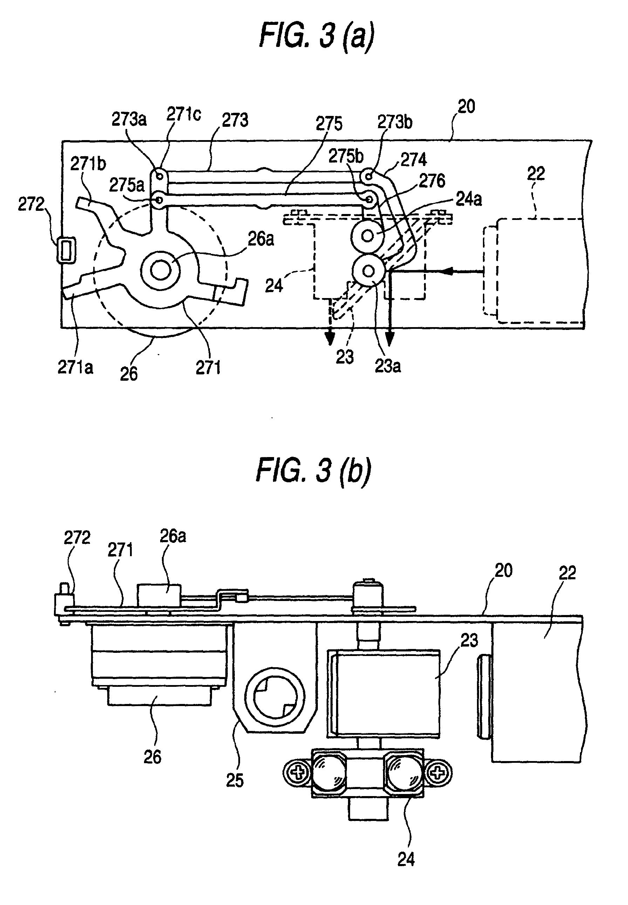

Each unit mounted on the tilt table 20 as mentioned earlier will be described referring to Fig. 3.

Fig. 3 is an enlarged view of the periphery of a mirror for panning. Fig. 3(a) is a top view of the periphery of a mirror for panning. Fig. 3(b) is a front view of the periphery of a mirror for panning.

A telephotograph camera 22 is arranged on the support plate 17 of the tilt table 20 so that the light axis (image pickup light axis) may be coaxial with the rotation axis of the axes 20a, 20b of the tilt table 20 and the image pickup direction of the telephotograph camera 22 may face the mirror for panning 23.

The mirror for panning 23 is arranged in front of the telephotograph camera 22. A light reflected on the mirror for panning 23 is entered to the telephotograph camera 22. That is, the image pickup direction of the telephotograph camera 22 is reflected by the mirror for panning 23 (bold arrow in Fig. 3(a)). The mirror for panning 23 is rotatable about the mirror axis 23a perpendicular to the image pickup light axis of the telephotograph camera 22. The mirror for panning 23 and the mirror axis 23a are fixed to each other.

The motor forpanning 26 for rotating the mirror for panning 23 is attached to the support plate 16 of the tilt table 20 and rotates the mirror for panning 23 via the link mechanism 27.

The range sensor 24 is also interlocked with the mirror for panning 23 by the motor for panning 26 via the link mechanism 27 and rotates about a sensor axis parallel with the mirror axis 23a. The range sensor 24 and the sensor axis 24a are fixed to each other.

High-accuracy distance measurement is made possible by orienting the range direction by the range sensor 24 (bold arrow in Fig. 3(a)) to the object and irradiating infrared rays.

The range sensor 24 is located below the mirror for panning 23 when seen in the front direction, as shown in Fig. 3(b).

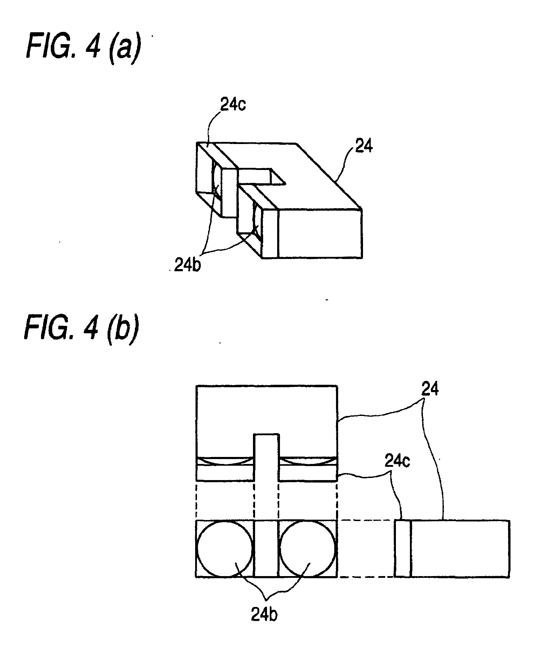

Configuration of the range sensor 24 will be described referring to Fig. 4.

Fig. 4 shows a perspective view and a three-view drawing of a range sensor. Fig. 4(a) is a perspective view of the range sensor. Fig. 4(b) is a three-view drawing of the range sensor.

The range sensor 24 has sensor lenses 24b and arranges a stray light prevention hood 24c on the sensor opening of the sensor lenses 24b. The stray light prevention hood 24c reduces the diffused reflection from a direction any diffused reflection light is not permitted on the range sensor 24. This prevents an error in ranging by the range sensor 24.

Referring to Fig. 3 again, the link mechanism 27 will be described.

The link mechanism 27 includes a motor link member 271, a stopper member 272, a first mirror link member 273, a second mirror link member 274, a first sensor link member 275, and a second sensor link member 276.

The motor link member 271 is fixed to a motor shaft 26a and rotates about the center axis of the motor shaft 26a in accordance with the rotation of the motor shaft 26a by a motor for panning 26. This rotation range (angle) is determined by either the first engaging section 271a and the second engaging section 271b of a motor link member 271 engaging a stopper member 272 fixed to the tilt table 20.

The motor link member 271 has a link piece 271c extending from the motor shaft 26a toward the periphery of the motor shaft. The link piece 271c engages a first mirror link member 273 via a first mirror pin 273a in a mutually rotatable fashion. The link piece 271c also engages a first sensor link member 275 via a first sensor pin 275a in a mutually rotatable fashion.

The first mirror link member 273 is a longitudinal member along the longitudinal direction of the tilt table 20. The end of the first mirror link member 273 (the telephotograph camera 22 side) opposite from the end engaging the first mirror pin 273a engages a second mirror link member 274 via a second mirror pin 273b in a mutually rotatable fashion.

Further, the second mirror link member 274 is fixed to the mirror axis 23a. Movement of the second mirror link member 274 allows the mirror for panning 23 to rotate about the mirror axis 23a.

The first sensor link member 275 is a longitudinal member along the longitudinal direction of the tilt table 20. The end of the first sensor link member 275 (the telephotograph camera 22 side) opposite from the end engaging the first sensor pin 275a engages a second sensor link member 276 via a second sensor pin 275b in a mutually rotatable fashion.

Further, the second sensor link member 276 is fixed to the sensor axis 24a. Movement of the second sensor link member 276 allows the range sensor 24 to rotate about the sensor axis 24a.

A rotation angle double that of the mirror for panning 23 is given to the range sensor 24, in accordance with the ratio of the distance between the motor shaft 26a and the axis of the first mirror pin 273a to the distance between the mirror axis 23a and the second mirror pin 273b and the ratio of the distance between the motor shaft 26a and the axis of the first sensor pin 275a to the distance between the sensor axis 24a and the second sensor pin 275b.

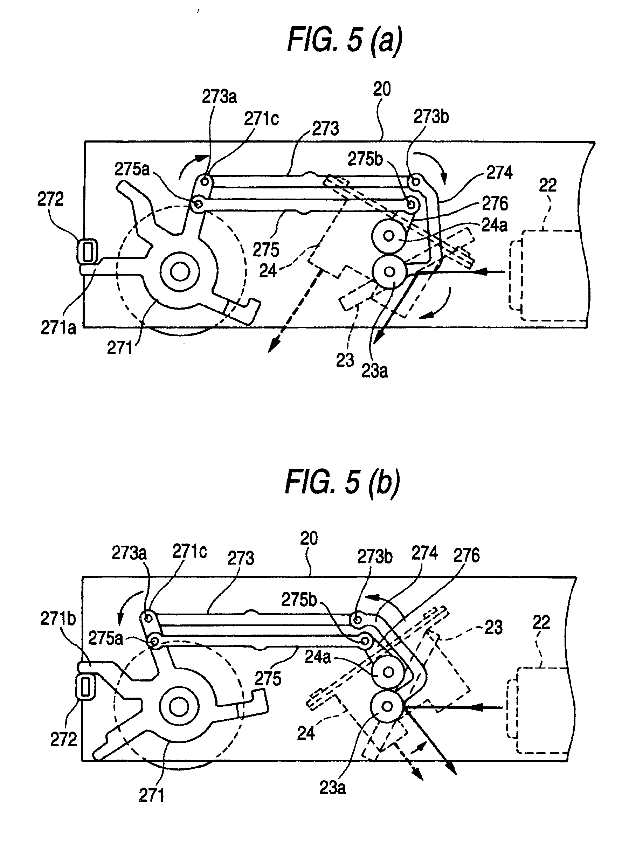

Operation of the link mechanism 27 will be described referring to Fig. 5. Fig. 5 shows the operation of a link mechanism. Fig. 5(a) shows the state the link mechanism has swiveled clockwise. Fig. 5(b) shows the state the link mechanism has swiveled counterclockwise.

Fig. 5(a) and Fig. 5(b) are top views of the periphery of the mirror for panning, same as Fig. 3(a).

### [Clockwise swivel]

As shown in Fig. 5(a), in case the link mechanism 27 has swiveled clockwise, the motor link member 271 swivels clockwise as long as the first engaging section 271a engages the stopper member 272.

The link piece 271c also swivels clockwise. The first mirror link member 273 travels clockwise (in the longitudinal direction of the tilt table 20 toward the telephotograph camera 22). This allows the second mirror link member 274 to travel thus swiveling the mirror axis 23a clockwise. This swivels the mirror for panning 23 clockwise and the image pickup direction is tilted leftward.

The first sensor link 275 travels clockwise at the same time the link piece 271c travels clockwise. This allows the second sensor link member 276 to travel also, thus swiveling the sensor axis 24a clockwise. The range sensor 24 also swivels clockwise and the range direction is tilted leftward.

### [Counterclockwise swivel]

As shown in Fig. 5(b), in case the link mechanism 27 has swiveled counterclockwise, the motor link member 271 swivels counterclockwise as long as the second engaging section 271b engages the stopper member 272.

The link piece 271c also swivels counterclockwise. The first mirror link member 273 travels counterclockwise (in the longitudinal direction of the tilt table 20 toward the stopper member 272). This allows the second mirror link member 274 to travel thus swiveling the mirror axis 23a counterclockwise. This swivels the mirror for panning 23 counterclockwise and the image pickup direction is tilted rightward.

The first sensor link 275 travels counterclockwise at the same time the link piece 271c travels counterclockwise. This allows the second sensor link member 276 to travel also, thus swiveling the sensor axis 24a counterclockwise. The range sensor 24 also swivels counterclockwise and the range direction is tilted rightward.

For both clockwise and counterclockwise swivels, the rotation angle of the range sensor 24 is double that of the mirror for panning 23. This allows the range sensor 24 to swivel clockwise and counterclockwise while the image pickup direction coincides with the range direction.

This above is the description on the configuration and operation of the link mechanism 27.

While the link mechanism 27 is configured as mentioned earlier, the link mechanism 27 may include a plurality of gears and/or pulleys for swivel operation as long as the image pickup direction is maintained the same as the range direction.

A wide angle camera 25 is arranged between the mirror for panning 23 and the motor for panning 26 and its optical axis is provided at a point crossing the rotation axis of the tilt table 20 in the same plane. Top views of Figs. 3 and 5 are not shown.

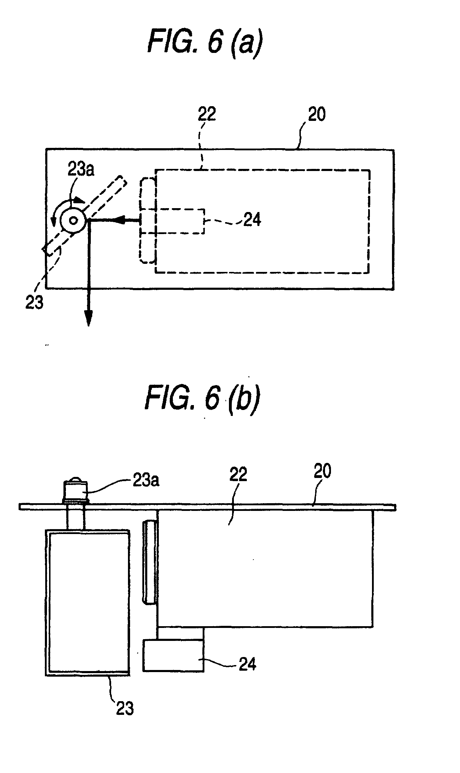

Referring to Fig. 6, a case where the range sensor 24 is provided just beneath the telephotograph camera 22 will be described.

Fig. 6 is an enlarged view of the periphery of a mirror for panning assumed when a range sensor is provided just beneath a telephotograph camera. Fig. 6(a) is a top view thereof. Fig. 6(b) is a front view thereof. In Fig. 6, the link mechanism for rotating the mirror axis 23a is not shown. Directly rotating the mirror axis 23a with a motor eliminates the need for the link mechanism 27.

The range optical axis of the range sensor 24 located just beneath the telephotograph camera 22 is in parallel with the image pickup optical axis of the telephotograph camera 22.

The mirror for panning 23 is arranged in front of the telephotograph camera 22. A light reflected on the mirror for panning 23 is entered to the telephotograph camera 22. That is, the image pickup direction of the telephotograph camera 22 is reflected by the mirror for panning 23 (bold arrow in Fig. 6(a)).

The mirror for panning 23 is also in front of the range sensor 24 so that the range direction is identical with the image pickup direction.

The mirror for panning 23 is rotatable about the mirror axis 23a perpendicular to the image pickup light axis of the telephotograph camera 22 and the range direction of the range sensor 24. The mirror for panning 23 and the mirror axis 23a are fixed to each other.

With the oblong mirror for panning 23, the image pickup direction of the telephotograph camera 22 and the range direction of the range sensor 24 can be tilted leftward and rightward (longitudinal direction of the tilt table 20) while both directions are maintained the same.

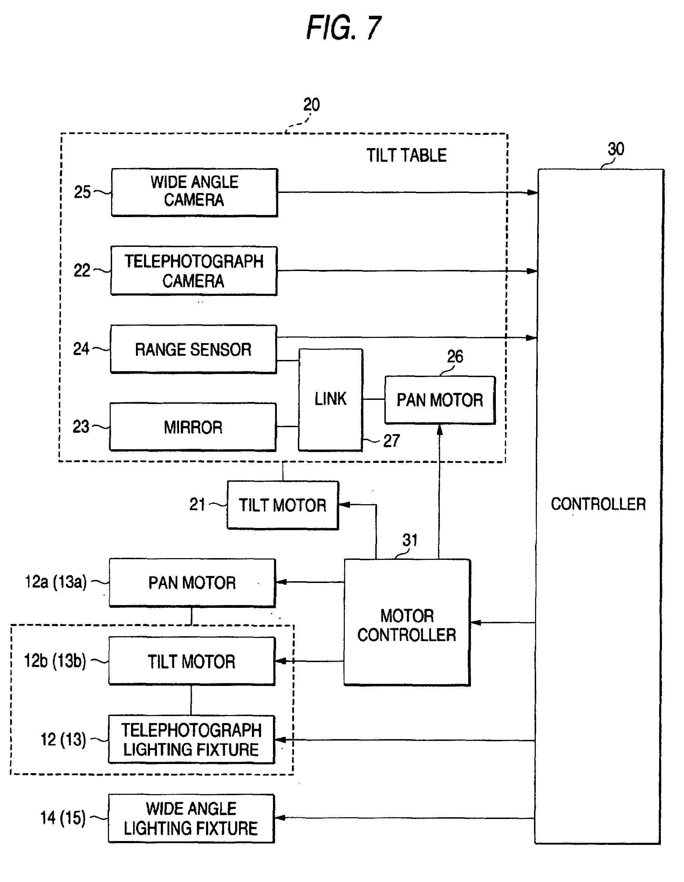

The circuit of the iris image pickup apparatus 10 will be described referring to Fig. 7.

Fig. 7 is a circuit diagram of the electric system of the iris image pickup apparatus.

An image picked up by a wide angle camera mounted on the tilt table 20 and a distance measured by the range sensor 24 mounted on the same are inputs of a controller 30 not shown in Fig. 1, and controls the driving of a pan motor 26 and a tilt motor 21 via a motor controller 31, as well as controls driving of a pan motor for lighting 12a (13a) and a tilt motor for lighting 12b (13b) to pick up the iris using the telephotograph camera 22.

The controller 30 also controls lighting and shutting off a iris lighting fixture 12 (13) and a lighting fixture for wide angle cameras 14 (15).

Next, operation of the iris image pickup apparatus of the foregoing configuration will be described.

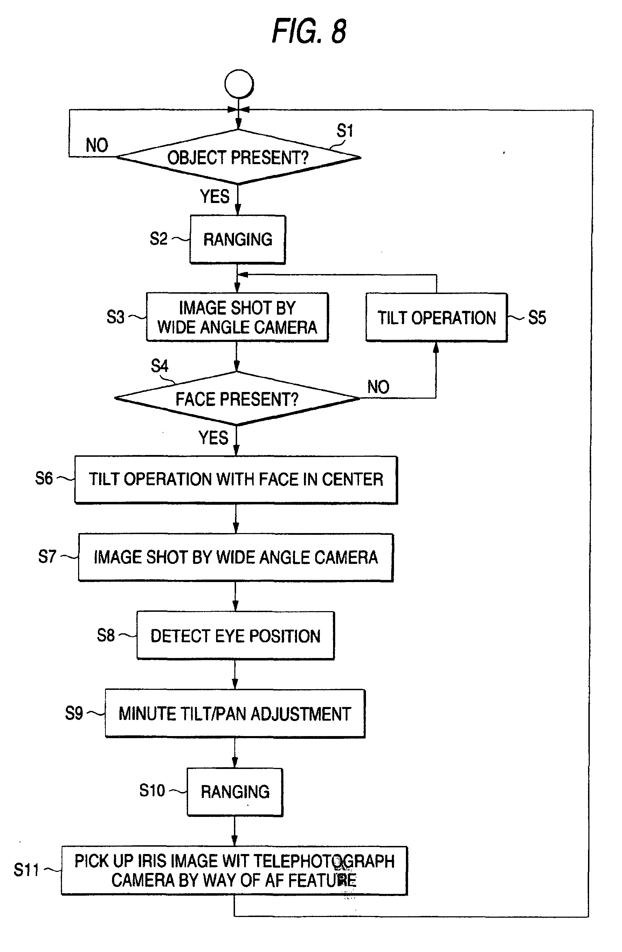

Fig. 8 is a flowchart showing the operating procedure for the iris image pickup apparatus. In step S1, the iris image pickup apparatus is the standby state waiting for an object (person to be authenticated) to enter a predetermined front range of the iris image pickup apparatus. In this standby state, each motor 21, 26, 12a, 13a, 12b, 13b is respectively at default position (home position) and the telephotograph camera 22 and the range sensor 24 face the default position also.

Default position for the telephotograph camera 22 and the range sensor 24 is approximately in the center of the maximum swivel angle direction or a predetermined direction.

The range sensor 24 emits infrared rays in the standby state or constantly or per predetermined time. The presence/absence of a resulting reflected light allows the controller 30 shown in Fig. 7 to determine the presence of an object. In case the object has entered the image pickup range, the distance to the object is measured based on the reflected light (step S2) and execution proceeds to step S3.

In step S3, images-are picked up by the wide angle camera 25. At this time, lighting fixtures for wide angle cameras 14, 15 are illuminated. In step S4, the controller 30 uses pattern matching to determine whether the face of the object is in the image picked up. In case the face of the object is not found, the controller 30 outputs a driving instruction to a motor for tilt 21 via a motor controller 31 to perform tilt operation (step S5), then captures the image picked up by the wide angle camera in step S3. The subsequent steps S3, S4, S5 are repeated until the whole face image is captured.

In case the face pattern is found in the image picked up by the wide angle camera, execution proceeds from step S4 to step S6. Tilt operation of the tilt table 20 is made so that the face comes in the center of the image picked up by the wide angle camera. Pan position and tilt position of the iris lighting fixtures 12, 13 in Fig. 1 are adjusted. The illuminating light irradiation direction is swiveled in advance so that the light illuminates the face. The mirror for panning 23 is swiveled in advance so that the telephotograph camera 22 can pick up the face. Corresponding accurate adjustment is made in step S9 as mentioned later. In step S7, an image is picked up by wide angle camera. Execution then proceeds to step S8.

In step S8, the controller 30 detects the position of the left eye or right eye from the image picked up by the wide angle camera captured in step S7. From the image picked up by the wide angle camera, the direction an eye is present is known, but the distance to the "eye" picked up is unknown. When the distance is unknown, a parallax in the right/left direction is present between the wide angle camera 25 and the telephotograph camera 22. Thus the position of the iris image in the screen is dislocated depending on the swivel angle of the mirror for panning 23. Because an aberration is present in the periphery of a camera lens, an iris image is preferably in the center of a picked up image to obtain an iris image with high authentication accuracy. Thus, the controller 30 also uses the measurement value of the range sensor 24 measured in step S2 to calculate the position of an eye to be picked up.

Next, the controller 30 converts the position of an eye obtained in step S8 to coordinates of the telephotograph camera 22. The controller 30 then obtains the accurate tilt position of the tilt table 20 and the accurate pan position of the mirror for panning 23 as well as the accurate tilt position and pan position of the iris lighting fixtures 12, 13, to carry out minute adjustment of the tile position and the pan position (step S9).

In step S10, the controller 30 uses the range sensor 24 to measure the distance to the iris. The range sensor 24 is interlocked with the pan swivel of the mirror for panning 23 and its range direction is adjusted upward and downward by the tilt table 20. Thus the infrared rays for measurement are irradiated toward the iris. Receiving a light reflected on a cheek below an eye for example allows accurate measurement of the distance to the iris.

In the next step S11, the distance to the iris measured in step S10 is preset to the telephotograph camera 22, a focus lens (not shown) is rapidly driven to focus on the distance. Subsequently, a general auto-focus (AF) technology is used to bring the iris in focus. For example, a focus lens driving motor is traveled step by step to obtain an image picked up by the telephotograph camera 22. A focus lens position where the high-frequency component in the image is highest is assumed as a focus. The image then obtained is acquired as an iris image to be authenticated. When the telephotograph camera 22 is used to pick up an iris image, the controller 30 illuminates either or both of the iris lighting fixtures 12, 13. The controller 30 then passes the iris image picked up at the focus to authentication apparatus (not shown). Execution returns to step 1.

While the invention has been described in detail referring to a specific embodiment, Those skilled in the art will appreciate that the invention may be modified or corrected in various forms without departing from the spirit and the range of the invention.

This application is based on the Japanese Patent Application No. 2000-315438 filed October 16, 2000, which is incorporated herein by reference.

### Industrial Applicability

According to this iris image pickup apparatus, by providing a range sensor capable of detecting the distance to an object, it is possible to measure the distance to an object with a single wide angle camera (conventional object person position recognition camera) . This reduces the manufacturing cost of an iris image pickup apparatus.

The range sensor is mounted on a tilt table and makes ranging while tilted upward or downward thus expanding upward and downward the range of measuring the distance to the object.

The image pickup direction of the telephotograph camera coincides with the range direction of the range sensor so that it is possible to use the distance to the object detected by the range sensor for adjusting the focus of the telephotograph camera.

## Claims

1. An iris image pickup apparatus for picking up the iris of an object comprising:
a wide angle camera for picking up an image of an object;
a range sensor for measuring the distance to said object;
a telephotograph camera for picking up the iris of said object based on the image picked up by said wide angle camera and the range measured by said range sensor; and
a tilt table where said telephotograph camera and said range sensor are mounted with the image pickup direction of said telephotograph camera for said iris approximately matching the range direction of said range sensor for said object, said tilt table tilting upward/downward about the tilt rotation axis;
wherein said range sensor measures the distance to said object with the tilting of said tilt table,
wherein said telephotograph camera picks up said iris of said object based on the image picked up by said wide angle camera and the range measured by said range sensor.

2. The iris image pickup apparatus according to claim 1, wherein said range sensor is positioned close to approximately beneath or above said telephotograph camera and that the range optical axis of said range sensor is parallel with the image pickup optical axis of said telephotograph camera.

3. The iris image pickup apparatus according to claim 1 or 2, further comprising swivel means for swiveling said image pickup direction by said telephotograph camera and said range direction by said range sensor to the direction approximately orthogonal to the operating direction of said tilt table.

4. The iris image pickup apparatus according to claim 3,
wherein said swivel means comprises a reflecting mirror for reflecting said image pickup direction by said telephotograph camera and said range direction by said range sensor,
wherein said iris image pickup apparatus swivels said image pickup direction and said range direction to a direction approximately orthogonal to the operating direction of said tilt table.

5. The iris image pickup apparatus according to claim 3 or 4, wherein said swivel means orients said range direction to an approximately center direction of the maximum swivel angle of said swivel means or to a predetermined direction while said range sensor is in standby state before detecting said object.

6. The iris image pickup apparatus according to claim 1 further comprising:
a reflecting mirror for reflecting said image pickup direction by said telephotograph camera; and
link means for rotating said telephotograph camera to a direction approximately orthogonal to the operating direction of said tilt table at a rotation angle double the rotating angle of said reflecting mirror.

7. The iris image pickup apparatus according to claim 6, wherein said link means orients said range direction to an approximately center direction of the maximum swivel angle of said swivel means or to a predetermined direction while said range sensor is in standby state before detecting said object.

8. The iris image pickup apparatus according to any one of claims 1 to 7, wherein said ranging sensor comprises a stray light prevention hood on the sensor opening of said range sensor.
